# EUROPEAN PATENT SPECIFICATION

(11) **EP 4 340 810 B1**
(45) Date of publication and mention of the grant of the patent: **10.09.2025**
(21) Application number: 22730154.6
(22) Date of filing: 19.05.2022
(51) Int. Cl.: A61K 9/00, G02C 7/04, A61F 9/00

(54) **THERAPEUTIC LENSES FOR USE AS OCULAR BANDAGE AND SIMULTANEOUSLY FOR THE ADMINISTRATION OF EYE DROPS**
THERAPEUTISCHE LINSEN ZUR VERWENDUNG ALS AUGENBANDAGE UND GLEICHZEITIG ZUR VERABREICHUNG VON AUGENTROPFEN
LENTILLES THÉRAPEUTIQUES DESTINÉES À ÊTRE UTILISÉES COMME PANSEMENT OCULAIRE ET SIMULTANÉMENT POUR L'ADMINISTRATION DE GOUTTES OPHTLMIQUES

(30) Priority: 20.05.2021 IT 202100013076
(43) Date of publication of application: 27.03.2024
(73) Proprietor: Eye Pharma SpA, 16148 Genova (GE) (IT)
(72) Inventor: CAMPORA, Giuseppe, 16032 Ruta di Camogli (IT)
(74) Representative: Longoni, Alessandra
(86) International application number: PCT/EP2022/063537
(87) International publication number: WO 2022/243416

(56) References cited:
- ES-A1- 2 366 839
- ALESSANDRA PULLIERO; ALDO PROFUMO; ALBERTO IZZOTTI; SERGIO CLAUDIO SACCA: "Release of Aloe vera Extracts from Therapeutic Lenses", APPLIED SCIENCES, vol. 10, no. 24, 18 December 2020 (2020-12-18), pages 9055, XP002805380

## Description

The present invention relates to therapeutic contact lenses, in particular contact lenses soaked with *Aloe vera,* for use as ocular bandage and for the administration of eye drops through them.

*Aloe vera* extracts have been used in therapy due to their antimicrobial, anti-inflammatory, and epithelia-regenerative properties.

Therapeutic contact lenses have analgesic and protective effect and they are particularly useful in the treatment of several ocular diseases and in the post-surgical management of patients because they act as physical bandage of the corneal surface. In case of ocular surgery or eye injuries, the use of therapeutic lenses protects the eye for the time needed to heal.

The prolonged use of contact lenses, however, may cause significant ocular complications due to microbial infections, particularly in case of poor hygienic conditions, smoke, etc. Moreover, eye medication with eye drops in the presence of contact lenses may result to be not fully effective because the eye drops cannot permeate or can scarsely permeate through the contact lenses.

Therapeutic contact lenses containing *Aloe vera* extracts have the advantage, compared to other therapeutic contact lenses, of preventing the ocular irritation which may occur with a prolonged use of contact lenses, thanks to the analgesic, soothing and epithelia-regenerative actions of the *Aloe vera* extracts.

Therapeutic lenses containing *Aloe vera* extracts are known. An example is represented by the lenses sold by the Applicant under the tradename REGENERA^{®}, based on Contaflex 75, a non-ionic hydrogel.

Pulliero et al. (Appl. Sci. 2020, 10, 9055) discloses ionic lenses made from Filcon IV 3, a copolymer of 2-hydroxyethylmethacrylate and glycerol methacrylate, soaked with *Aloe vera* extract, which contains boric buffer, EDTA, 0.0002% polyhexanide, and poloxamer and polyvinylpyrrolidone, as surfactant agents.

We have now found that ionic therapeutic contact lenses containing *Aloe vera* extracts have the advantage to allow the passage of medicated eye drops and therefore they can be used as ocular bandage which does not need to be removed for the medication of the eye with eye drops. Therefore, the use of ionic therapeutic contact lenses containing *Aloe vera* extracts allows to adapt themselves to any therapy the patient is undergoing and/or needs in the post-surgery period.

Therefore, object of the present invention are ionic therapeutic contact lenses containing *Aloe vera* extracts for use as ocular bandage and for the contemporaneous administration of eye drops through them, without the need to remove them from the eye, for use in the administration of anti-inflammatory and/or antibiotic eye drops.

The therapeutic contact lenses for the use according to the present invention are particularly useful and advantageous for post-surgical therapeutic indications, for example after surgical operations such as trabeculectomy (glaucoma surgery), keratoconus (post cross-linking), refractive surgery (PRK), corneal transplantation, pterygium surgery, suturless vitrectomy, but without being limited to them.

They can be also useful and advantageous for ophthalmic non-surgical indications such as, but not limited to, corneal injuries and abrasions, keratitis, ulcers, diseases and dystrofies of the ocular surface, healing of the corneal epithelium.

For the use according to the present invention, the ionic therapeutic contact lenses containing *Aloe vera* extracts are preferably contact lenses made of a co-polymer of 2-hydroxyethyl methacrylate (2-HEMA) and methacrylic acid cross-linked with ethylenglycol dimethacrylate (EGDMA) plus an initiator. Preferably, the contact lenses have a water content of about 72% and a refractive index of 1.384.

Preferably, the *Aloe vera* extract contained in the therapeutic contact lenses for the use according to the present invention is in the form of a solution containing boric acid buffer, EDTA, 0.0002% polyhexanide and polyvinylpyrrolidone.

The therapeutic lenses containing the *Aloe vera* extract commercially known as Alvera^{®} are particularly preferred.

The ionic therapeutic contact lenses containing *Aloe vera* extracts, herein after also simply referred to as therapeutic contact lenses according to the invention, are worn day and night for a period not longer than 30 days, tipically for a period of 7-10 days, for treating the ocular surface in need of healing and/or as post-surgical ocular bandage.

While the therapeutic contact lenses according to the invention are worn, they exert an analgesic, rigenerative, anti-inflammatory and protective action. Advantageously, during the same period of time, the patient can be medicated by directly administering eye drops, without removing the contact lenses, because the therapeutic contact lenses according to the invention are permeable to the drug present in the eye drops and do not affect the therapeutic efficacy of the eye drops. The permeability of the contact lenses according to the present invention further ensures an improved administration of the eye drops, by increasing the bioavailability.

In principle, any anti-inflammatory eyedrops (with steroidal as well as non-steroidal anti-inflammatory drugs) and/or antibiotic eye drops can be administered in the presence of the therapeutic contact lenses according to the invention, provided that it is an aqueous solution and it is compatible with the material of the contact lenses.

Eye drops containing cortisonic drugs such as betamethasone or dexamethasone, anti-inflammatory drugs such as bromphenac or antibiotic drugs such as chloramphenicol are particularly preferred since they are usually used in post-surgical therapies.

The permeability of different eye drops through therapeutic contact lenses according to the invention has been experimentally assessed *in vitro* by HPLC quantification of the amount of drug permeated over time.

In specific comparison examples, the permeability of the drug through the therapeutic contact lenses according to the invention showed to be significantly different from that of other types of contact lenses, notably compared to non-ionic contact lenses and silicone contact lenses.

In the specific case of antibiotic eye drops, it has been also confirmed that the antibiotic efficacy of the drug remained unchanged after the passage through therapeutic contact lenses according to the invention.

The use of the therapeutic contact lenses according to the present invention is particularly useful and advantageous for the treatment of several ophthalmic diseases or in the post-surgical treatment because allows to optimize and personilize the treatment with ophthalmic eye drops while maintaining the ocular bandage.

### DESCRIPTION OF THE FIGURES

Fig. 1 - HPLC analysis of the drugs
Fig. 2 - interactions of the time course of the drugs through the lenses
Fig. 3 - average amounts of drugs passing through the lenses
Fig. 4 - inhibition of *Salmonella typhimurium* growth by chloramphenicol

### Materials and methods

Three different types of lenses were tested: (1) Contaflex 75 hydrogel non-ionic lenses containing *Aloe vera,* (2) hydrogel ionic lenses containing *Aloe vera* according to the present invention and (3) Purevision Belafilcon A silicon hydrogel lenses. The water content in the lenses is 75% for the non-ionic lenses and 72% for the ionic lenses, respectively, while the silicon lenses have a 36% water content. The material for the ionic lenses is a co-polymer of 2-hydroxyethyl methacrylate (2-HEMA) and methacrylic acid cross-linked with ethylenglycol dimethacrylate (EGDMA) plus an initiator.

### Pharmacological eye drops

Commercially available anti-inflammatory eye drops containing 0.1% dexamethasone, 0.2% betamethasone or 0.9 mg/ml bromphenac were used. The used antibiotic eye drops included 0.5% chloramphenicol. The interaction of these eye drops with the different types of therapeutic lenses was evaluated at different times (30 minutes, 1, 2 and 24 hours).

### HPLC gradient analysis

Dexamethasone, betamethasone, bromophenac and chloramphenicol contained in the respective eye drops were quantified in the liquid phase upstream and downstream, and their interaction with the lenses was evaluated with HPLC gradient analysis. The separation was carried out using an HPLC system consisting of a vacuum degasser, an autosampler, a capillary pump and a thermostated column compartment (Agilent series 1200, Agilent Technology, Palo Alto, CA, USA). Briefly, five microliters of sample were injected onto a 1.0 mm x 150 mm, 3.5 µm particle size, Symmetry300^{™} column (Waters). Mobile phase A was 0.1% formic acid in water and mobile phase B was 0.1% formic acid in acetonitrile. The flow rate was 20 µL/min and the elution was performed in this sequence: isocratic 90% A for 10 min, a linear gradient over the course of 50 min to 100% B maintained at 100% B for 15 min and finally a linear gradient to 90% A in 5 min. The re-equilibration time in 90% A was 20 min. The eluent flow was directly sent to the electrospray (ESI) ion source of the 6210 time-of-flight mass spectrometer (Agilent Techonology) to characterize the HPLC peaks. The following operation parameters were applied: capillary voltage: 4300 V; nebulizer pressure: 20 psig; drying gas: 5 L/min; gas temperature: 300°C; fragmented voltage: 250 V; skimmer voltage: 80 V; octapole RF: 250 V. The full-scan data, recorded using Agilent Technology's Mass Hunter software, were processed with Mass Hunter Qualitative Analysis (Agilent Technology).

The three types of lenses were tested by incubating the pharmacological compounds together for different times (30 min, 1, 2 and 24 hours). They were stratified over the lenses, which act as a filter, spotting 100 µL of eye drops on the upstream phase and 50 µL of physiological solution in the downstream phase. The areas of the peaks of the compounds normalized by volume were measured.

### Bacterial proliferation assay

The influence of lens interaction on the antibacterial effect of chloramphenicol was tested with a bacterial challenge using *Salmonella typhimurium* seeding 2 x 10⁹/mL bacteria on agar-coated plates. An antibiotic disk was inserted in the middle of the plate to verify the inhibition halo around the dish after 24 hours. Both the chloramphenicol eye drops and the liquid suspending the therapeutic lenses for storage were tested.

### Results

### HPLC gradient analysis

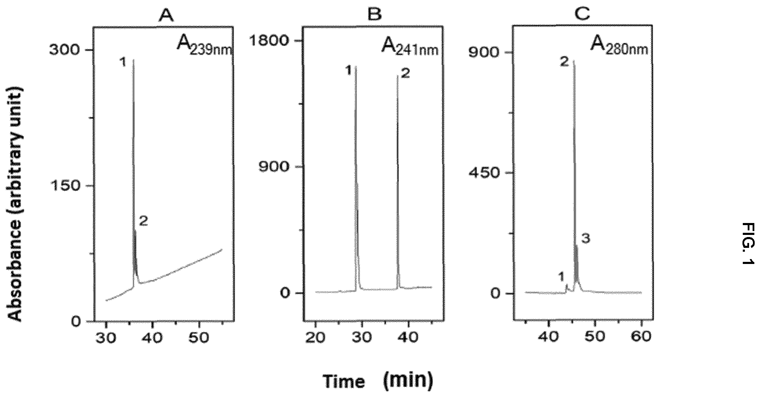

HPLC gradient analysis detected drugs in the aqueous solution well (Figure 1). For each tested drug, the specific elution times through the chromatographic column were as follows: dexamethasone 36 min, chloramphenicol 29 min, betamethasone 38 min and bromphenac 46 min. For each tested drug, the specific absorbance intensities were as follows: dexamethasone 239 nm, chloramphenicol and betamethasone 241 nm, bromphenac 280 nm.

### Time course and drug interaction with the lenses

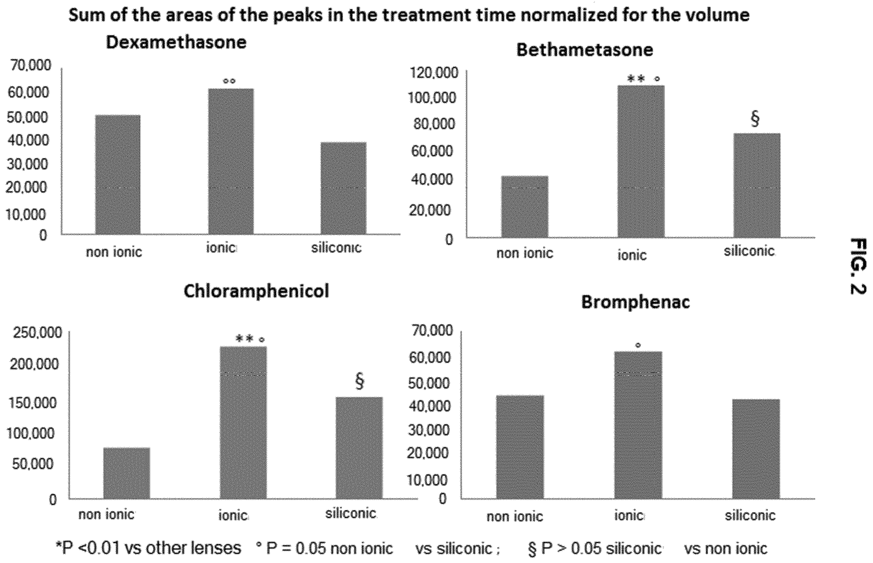

The time course interactions of the tested drugs through the lenses are reported in Figure 2. The time course was evaluated at 30 min, 1, 2 and 24 hours. The amount of drugs eluted through the lenses at each time course were determined by gradient HPLC as applied to the fluid eluted through the lenses contained in the lower parts of the wells.

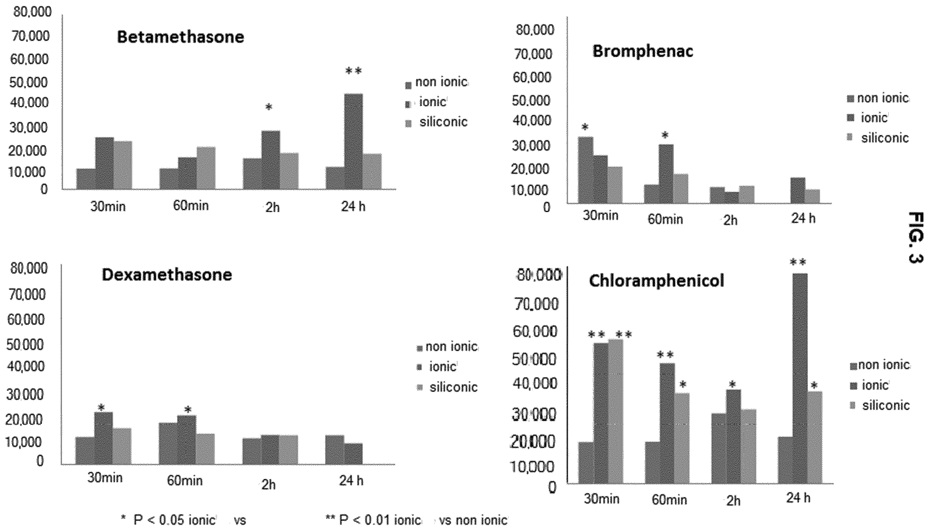

On the whole, the ionic therapeutic contact lenses according to the invention allows a better passage of all the tested drugs than the other contact lenses in term of amounts and/or time (Figure 3).

### Anti-bacterial capacity of chloramphenicol and passage through the lenses

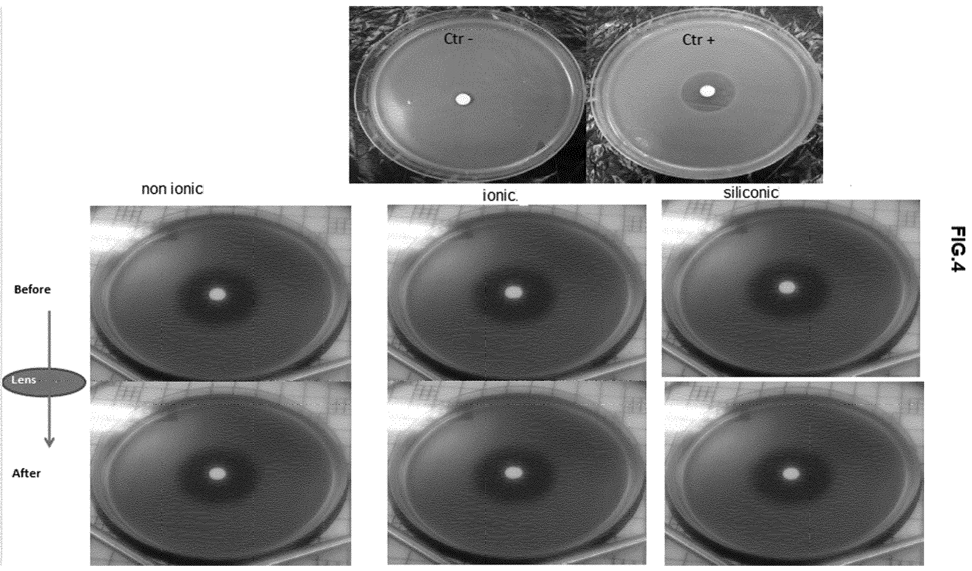

The antibacterial capacity of chloramphenicol was comparatively tested before and after the passage through the ionic therapeutic contact lenses according to the invention. The obtained results are reported in Figure 4. The diameter of the clear halo around the chloramphenicol depot area (central white spot) was not different. This result indicates that the passage through the lenses does not decrease the antibacterial capacity of chloramphenicol.

The obtained results have clinical relevance because allow a personalized medical approach since the ionic therapeutic contact lenses object of the present invention suit the therapy administered to each patient. This allows also to optimize the drug therapy in the treatment of the ocular surface.

## Claims

1. Ionic therapeutic contact lenses containing *Aloe vera* extract for use as ocular bandage and for the simultaneous administration of eye drops through them, without the need of removing them from the eyes, for use in the administration of anti-inflammatory and/or antibiotic eye drops.

2. The ionic therapeutic contact lenses for use according to claim 1 made of a material consisting of a copolymer of 2-hydroxyethylmethacrylate and methacrylic acid crosslinked with ethylene glycol dimethacrylate plus an initiator.

3. The ionic therapeutic contact lenses for use according to claim 1 or 2 with a water content of about 72% and a refractive index of 1.384.

4. The ionic therapeutic contact lenses for use according to anyone of the preceding claims wherein the *Aloe vera* extract is in the form of a solution containing boric acid buffer, EDTA, 0.0002% polyhexanide, poloxamer and polyvinylpyrrolidone.

5. The ionic therapeutic contact lenses for use according to anyone of the preceding claims for use in the administration of anti-inflammatory eye drops containing betamethasone, dexamethasone or bromphenac.

6. The ionic therapeutic contact lenses for use according to anyone of the preceding claims for use in the administration of antibiotic eye drops containing chloramphenicol.

7. The ionic therapeutic contact lenses for use according to anyone of the preceding claims for post-surgical therapeutic indications.

8. The ionic therapeutic contact lenses for use according to anyone of claims 1-6 for non-surgical therapeutic indications.

## Patentansprüche

1. Ionische therapeutische Kontaktlinsen, die *Aloe-Vera*-Extrakt enthalten, zur Verwendung als Augenverband und zur gleichzeitigen Verabreichung von Augentropfen durch sie, ohne dass sie aus den Augen entfernt werden müssen, zur Verwendung bei der Verabreichung von entzündungshemmenden und/oder antibiotischen Augentropfen.

2. Ionische therapeutische Kontaktlinsen zur Verwendung nach Anspruch 1, hergestellt aus einem Material, das aus einem Copolymer von 2-Hydroxyethylmethacrylat und Methacrylsäure besteht, vernetzt mit Ethylenglykoldimethacrylat, plus einem Initiator.

3. Ionische therapeutische Kontaktlinsen zur Verwendung nach Anspruch 1 oder 2 mit einem Wassergehalt von etwa 72 % und einem Brechungsindex von 1,384.

4. Ionische therapeutische Kontaktlinsen zur Verwendung nach einem der vorhergehenden Ansprüche, wobei der *Aloe-Vera*-Extrakt in der Form einer Lösung vorliegt, die Borsäurepuffer, EDTA, 0,0002 % Polyhexanid, Poloxamer und Polyvinylpyrrolidon enthält.

5. Ionische therapeutische Kontaktlinsen zur Verwendung nach einem der vorhergehenden Ansprüche zur Verwendung bei der Verabreichung von entzündungshemmenden Augentropfen, die Betamethason, Dexamethason oder Bromphenac enthalten.

6. Ionische therapeutische Kontaktlinsen zur Verwendung nach einem der vorhergehenden Ansprüche zur Verwendung bei der Verabreichung von antibiotischen Augentropfen, die Chloramphenicol enthalten.

7. Ionische therapeutische Kontaktlinsen zur Verwendung nach einem der vorhergehenden Ansprüche für postoperative therapeutische Indikationen.

8. Ionische therapeutische Kontaktlinsen zur Verwendung nach einem der Ansprüche 1-6 für nichtchirurgische therapeutische Indikationen.

## Revendications

1. Lentilles de contact thérapeutiques ioniques contenant de l'extrait *d'Aloe vera* destinées à être utilisées comme pansement oculaire et permettant l'administration simultanée de gouttes ophtalmiques à travers elles, sans qu'il ne soit nécessaire de les retirer des yeux, destinées à être utilisées dans l'administration de gouttes ophtalmiques anti-inflammatoires et/ou antibiotiques.

2. Lentilles de contact thérapeutiques ioniques destinées à être utilisées selon la revendication 1, fabriquées en un matériau constitué d'un copolymère de 2-hydroxyéthylméthacrylate et d'acide méthacrylique réticulé avec du diméthacrylate d'éthylène glycol plus un initiateur.

3. Lentilles de contact thérapeutiques ioniques destinées à être utilisées selon la revendication 1 ou 2, présentant une teneur en eau d'environ 72 % et un indice de réfraction de 1,384.

4. Lentilles de contact thérapeutiques ioniques destinées à être utilisées selon l'une quelconque des revendications précédentes, ledit extrait *d'Aloe vera* se présentant sous la forme d'une solution contenant un tampon d'acide borique, de l'EDTA, du polyhexanide à 0,0002 %, du poloxamère et de la polyvinylpyrrolidone.

5. Lentilles de contact thérapeutiques ioniques destinées à être utilisées selon l'une quelconque des revendications précédentes, destinées à être utilisées dans l'administration de gouttes ophtalmiques anti-inflammatoires contenant de la bétaméthasone, de la dexaméthasone ou du bromphénac.

6. Lentilles de contact thérapeutiques ioniques destinées à être utilisées selon l'une quelconque des revendications précédentes, destinées à être utilisées dans l'administration de gouttes ophtalmiques antibiotiques contenant du chloramphénicol.

7. Lentilles de contact thérapeutiques ioniques destinées à être utilisées selon l'une quelconque des revendications précédentes, pour des indications thérapeutiques post-chirurgicales.

8. Lentilles de contact thérapeutiques ioniques destinées à être utilisées selon l'une quelconque des revendications 1 à 6, pour des indications thérapeutiques non chirurgicales.
